# EUROPEAN PATENT APPLICATION

(11) **EP 0 526 413 A2**
(43) Date of publication of application: **03.02.1993**
(21) Application number: 92830344.5
(22) Date of filing: 01.07.1992
(51) Int. Cl.: B09B 3/00

(54) **Ecologically acceptable composition for desiccation of solid, semi-solid and liquid waste**

(30) Priority: 04.07.1991 IT RM910487
(71) Applicant: Bazzardi, Paola Patrizia, I-00197 Roma RM (IT); Boidi, Stefano, I-00192 Roma RM (IT); Caglioti, Luciano, I-00197 Roma RM (IT); Sbarigia, Giancarlo, I-00162 Roma RM (IT)
(72) Inventor: Bazzardi, Paola Patrizia, I-00197 Roma RM (IT); Boidi, Stefano, I-00192 Roma RM (IT); Caglioti, Luciano, I-00197 Roma RM (IT); Sbarigia, Giancarlo, I-00162 Roma RM (IT)
(74) Representative: Di Cerbo, Mario

(57) **Abstract**

The invention relates to a composition which is particularly suited for desiccation of solid, semisolid and liquid waste produced by the human and animal metabolism at whatever stage of its development. The composition is characterized in that it consists essentially of the following components:
15-40% by weight of a base substance chosen from the group comprising poliolefines, acrylic based polymers or copolymers, and starch polymers with mixed sodium and aluminium salts of 2-propenammide-co-2-propenoic acid and combinations thereof;
20-75% by weight of a binder/adjuvant chosen from the group comprising cement, calcium sulphate, salts and oxides of metals of the second, third and fourth group of the periodic system and combinations thereof; and, optionally,
10-30% by weight of substances chosen from the group comprising disinfectants, desiccants, absorbers, odorizers, colorants and combinations thereof.

## Description

The present invention has as its subject an ecologically acceptable compound for desiccation of solid, semi-solid and liquid waste, in particular of waste produced by the human and animal metabolism at whatever stage of its development.

As it is known, in surroundings frequented by newborn infants, old people, sick people or animals, there arises the problem of how to eliminate - for both hygienic and aesthetic reasons - the involuntary emission of food and drink from the mouth (vomit) or the involuntary expulsion from the intestine of the undigested remains of food. This problem appears in a particularly frequent and serious manner in hospitals, in vehicles for transport on land, sea.and air, and in the home.

In the past, in order to solve the above mentioned problem, it was necessary to resort to mechanical removal of the residue, or alternatively to expedients which were not entirely suitable, such as the use of sand or sawdust (a collection of minute fragments of wood which are produced during the operation of sawing wood). In this case, in fact, there are disadvantages both during the treatment stage and during removal of the material to be treated. During treatment, the disadvantages are mainly due to the fact that there is practically no desiccant action, to the fact that the physical transformation of the substrate is inefficient, and to the fact that it is necessary to use large amounts of absorbent. During removal, the most serious disadvantage is connected with the fact said removal required mechanical intervention which is exacting, unpleasant and insufficient from a hygienic point of view. In this case there is also the problem that the material produced cannot easily undergo further treatment.

The composition according to the present invention allows all the above mentioned disadvantages to be overcome, furthermore offering additional advantages which will be clearly seen from the following description.

The composition according to the present invention is characterized in that it consists essentially of the following components:
15-40% by weight of a base substance chosen from the group comprising poliolefines, acrylic based polymers or copolymers, and starch polymers with mixed sodium and aluminium salts of 2-propenammide-co-2-propenoic acid and combinations thereof;
20-75% by weight of a binder/adjuvant chosen from the group comprising cement, calcium sulphate, salts and oxides of metals of the second, third and fourth group of the periodic system of the elements and combinations thereof; and, optionally,
10-30% by weight of substances chosen from the group comprising disinfectants, desiccants, absorbers, odorizers, colorants and combinations thereof.

The base substance can be at least a starch copolymer (C₆H₁₀O₅)ₙH₂O containing grafted side chains consisting in mixed sodium and aluminium salts (C₃H₃O₂Na)ₙ and [(C₃H₃O₂)₃Al]ₙ of 2-propenammide (C₃H₅NO)ₙ-co-2 -propenoic acid.

In particular, the binder/adjuvant can be chosen from the group consisting of calcium sulphate, magnesium sulphate, calcium oxide, magnesium oxide, silica, alumina and combinations thereof.

In the composition according to the present invention the cement, at least in part, can be quick-drying cement.

The main advantages provided by use of the compound according to the present invention can be summed up as follows:
- extremely efficient desiccant action;
- physical transformation of the substrate to aid in its removal;
- use of small quantities of absorbent;
- non-exacting mechanical removal with results which are more than satisfactory from a hygienic point of view;
- total harmlessness of the product
- completeness of the operations as a whole
- easy handling of the product
- notable reduction in volume of the resulting material to be treated
- great limitation and reduction of the unpleasant aspects of the operations.

Up to this point a general description of the composition of the present invention has been given. With the aid of the following example, which refers to a specific embodiment of the invention, a more detailed description of its objects, characteristics, advantages and method of application will now be given.

### EXAMPLE

### Use of the composition for treatment of vomit

On the traces of vomit left by an elderly person bedridden in hospital, 50 g of a mixture having the following composition were poured:
33% by weight of starch copolymer (C₆H₁₀O₅)ₙH₂O containing grafted side chains consisting in mixed sodium and aluminium salts (C₃H₃O₂Na)ₙ and [(C₃H₃O₂)₃Al]ₙ of 2-propenammide (C₃H₅NO)ₙ-co-2-propenoic acid;
33% of calcium sulphate and 33% of cement.

Within 5 minutes the product completely absorbs said traces, which are transformed into a semisolid substance which can easily be removed.

## Claims

1. A composition characterized by the fact that it consists essentially of the following components:
15-40% by weight of a base substance chosen from the group comprising poliolefines, acrylic based polymers or copolymers, and starch polymers with mixed sodium and aluminium salts of 2-propenammide-co-2-propenoic acid and combinations thereof;
20-75% by weight of a binder/adjuvant chosen from the group comprising cement, calcium sulphate, salts and oxides of metals of the II, III and IV group of the periodic system of the elements and combinations thereof; and, optionally, 10-30% by weight of substances chosen from the group comprising disinfectants, desiccants, absorbers, odorizers, colorants and combinations thereof.

2. The composition according to claim 1, in which the base substance consists of at least one starch copolymer (C₆H₁₀O₅)ₙH₂O containing grafted side chains consisting in mixed sodium and aluminium salts (C₃H₃O₂Na)ₙ and [(C₃H₃O₂)₃Al]ₙ of 2-propenammide (C₃H₅NO)ₙ-co-2-propenoic acid.

3. The composition according to claim 1 or 2, in which the binder/adjuvant is chosen from the group comprising calcium sulphate, magnesium sulphate, calcium oxide, magnesium oxide, silica, alumina and combinations thereof.

4. The composition according to any one of the preceding claims, in which, at least in part, the cement is quick-drying cement.
